# EUROPÄISCHE PATENTSCHRIFT

(11) **EP 2 964 309 B1**
(45) Veröffentlichungstag und Bekanntmachung des Hinweises auf die Patenterteilung: **10.10.2018**
(21) Anmeldenummer: 13714347.5
(22) Anmeldetag: 25.02.2013
(51) Int. Cl.: A61M 25/06, A61M 25/00

(54) **KATHETERPUNKTIONSBESTECK**
CATHETER PUNCTURING DEVICE
ENSEMBLE DE PONCTION POUR CATHÉTER

(43) Veröffentlichungstag der Anmeldung: 13.01.2016
(73) Patentinhaber: Tietze, Bernd, 91325 Adelsdorf (DE)
(72) Erfinder: Tietze, Bernd, 91325 Adelsdorf (DE)
(74) Vertreter: Kodron, Felix
(86) Internationale Anmeldenummer: PCT/IB2013/051516
(87) Internationale Veröffentlichungsnummer: WO 2014/128535

(56) Entgegenhaltungen:
- EP-A1- 2 127 692
- WO-A1-02/053045
- WO-A1-2010/111283
- DE-A1- 2 340 534

## Beschreibung

Die Erfindung betrifft ein Katheterpunktionsbesteck gemäß dem Oberbegriff des Anspruchs 1.

Ist es zur Versorgung eines Patienten in stationärer Behandlung notwendig, einen Katheter zu legen, so erfolgt dies durch das medizinische Personal in der Regel nach der sogenannten "Seldinger"-Methode. Dabei wird in einem ersten Schritt das Gefäß (häufig eine Arterie oder Vene), in das der Katheter einzuführen ist, mit einer hohlen Punktionsnadel punktiert. Danach wird durch die hohle Punktionsnadel in das Gefäß ein Führungsdraht eingeschoben. Die Hohlnadel wird anschließend über den innen liegenden Führungsdraht wieder zurückgezogen, so dass der Führungsdraht weiter in das punktierte Gefäß vorgeschoben werden kann um dieses unter Verwendung eines über den Führungsdraht geschobenen Dilatators zunächst etwas aufzuweiten. Nach Entfernen des Dilatators wird der eigentliche Katheter über den liegenden Führungsdraht in das Gefäß eingeschoben und in die gewünschte Endstellung verschoben. Als letzter Schritt wird der Führungsdraht durch den Katheter wieder vorsichtig herausgezogen.

Diese Methode zur Punktion von Blutgefäßen nach Seldinger zum Zweck der Katheterisierung ist zwar eine Standardmethode, aber zum Legen des Katheters sind hierbei eine Vielzahl verschiedener Handgriffe und Instrumente erforderlich, die eine große Erfahrung in diesem Bereich voraussetzen. Zudem besteht aufgrund des mehrfachen Einschiebens und Herausziehens verschiedener Gegenstände die Gefahr, dass es zu Verunreinigungen von Gegenstände oder Personen durch austretendes Blut kommt. Schließlich ist auch die Gefahr einer Luftembolie gegeben, wenn nämlich ein leichter Unterdruck im punktierten Gefäß herrscht. Es sind daher Katheterpunktionsbestecke als Hilfsmittel zur Katheterisierung bekannt, die eine vereinfachte Handhabung bewirken sollen.

Aus der Veröffentlichung DE 2340534 A1 ist eine Vorrichtung zum Einführen eines Venenkatheters bekannt mit einem in eine Venenöffnung einführbaren Führungsschlauch, der einen zur Aufnahme des Katheterschlauches bemessenen Innenkanal aufweist und in Längsrichtung auftrennbar ist, um vom eingeführten Katheter entfernt werden zu können, wobei der Führungsschlauch für den Katheterschlauch mittels einer hosenartigen Verbindungsstelle mit einem zweiten Führungsschlauch für eine Punktiernadel zu einem gemeinsamen Führungsabschnitt vereinigt ist.

Aus der DE 101 00 102, ebenfalls veröffentlicht als WO 02/053045 A1, ist ebenfalls ein Katheterpunktionsbesteck bekannt, welches das Legen eines Katheters vereinfacht, indem es die für eine Punktion und das Legen eines Katheters erforderlichen Utensilien in einem aus mehreren Teilen zusammengeführten System bereitstellt und dabei ohne einen Führungsdraht oder zusätzliche Dilatationsgeräte auskommt.

Das dort offenbarte Katheterpunktionsbesteck umfasst dabei ein rohrartiges Gehäuse mit einem länglichen Gehäuseabschnitt, der in einen Verlängerungsabschnitt übergeht, und von dem unter einem Winkel ein Abzweigabschnitt abzweigt. Der Gehäuseabschnitt und der Abzweigabschnitt weisen eine durchgehende seitliche Öffnung auf, die von einem sich längs des Abzweigabschnitts und des Gehäuseabschnitts erstreckenden, flexiblen Mantel verschlossen wird, in den ein Katheter einschiebbar ist. Die sich längs des länglichen Gehäuseabschnitts und des Verlängerungsabschnitts erstreckende Punktionsnadel tritt bei der Punktion des Gefäßes aus der Spitze des Gehäuses aus und wird nach erfolgter Punktion in das Gehäuse bis hinter die Abzweigstelle des Abzweigabschnitts zurückgezogen. Am hinteren Ende der Punktionsnadel befindet sich ein Blutauffangbehälter.

Anschließend wird der Katheter über den Abzweigabschnitt und den Gehäuseabschnitt aus dem Gehäuse durch den flexiblen Mantel in das punktierte Gefäß eingeführt bis zur gewünschten Tiefe. Erst dann wird das Katheterpunktionsbesteck aus dem punktierten Gefäß des Patienten herausgezogen. Zuletzt wird der flexible Mantel aus dem Gehäuse herausgezogen bzw. über einen seitlichen Längsschlitz vom Katheter abgeschält. Insgesamt liegen also bei dem offenbarten Katheterpunktionsbesteck nach der DE 101 00 102 nach erfolgter Punktion neben dem gelegten Katheter zwei Teile vor, zum einen das Gehäuse mit der Punktionsnadel und dem Blutauffangbehälter, zum anderen der abgelöste Mantel.

Es sind neben diesem geschilderten Stand der Technik noch weitere Methoden der Katheterlegung nach modifizierter Seldingertechnik bekannt, wobei einige der stets mehrteiligen Katheterpunktionsbestecke mit der sog. "Peel-Away"-Technik arbeiten, bei welcher sich die Einführhülsen nach erfolgter Katheterplatzierung im punktierten Gefäß an einer "Peel-Away-Schleuse" in zwei Teile teilen und aus dem Gefäß ohne eine Bewegung des Katheters herausgezogen werden können.

Es ist vor diesem Hintergrund die Aufgabe der vorliegenden Erfindung, ein Katheterpunktionsbesteck zu schaffen, welches sicherstellt, dass die Punktionsnadel nach der Rückführung in das Gehäuse des Katheterpunktionsbesteckes dort auf konstruktiv einfache und nicht reversible weise gesichert wird. Auf diese Weise soll eine evtl. Infektion an dieser mit Patientenblut benetzten Punktionsnadel sicher ausgeschlossen werden.

Erreicht wird dies durch ein Katheterpunktionsbesteck mit den kennzeichnenden Merkmalen des Anspruch 1.

Die Unteransprüche haben vorteilhafte Ausgestaltungen der Erfindung zum Gegenstand.

Das erfindungsgemäße Katheterpunktionsbesteck stellt ein geschlossenes, einteiliges System dar, welches alle für die Punktion notwendige Bauteile in einem Gehäuse vereint und zur Verfügung stellt und gleichzeitig ermöglicht, den Katheter nach erfolgter Einführung in das punktierte Gefäß mit einem Handgriff aus dem Katheterpunktionsbesteck zu lösen und freizulegen.

Um es nun weiterzubilden ist das erfindungsgemäße Katheterpunktionsbesteck-Gehäuse einteilig ausgebildet, wobei der längliche, rohrförmig geschlossene Gehäuseabschnitt sowohl eine mit ihrer Spitze aus dem länglichen Gehäuseabschnitt austretende, sich längs des länglichen Gehäuseabschnitts und des Verlängerungsabschnitts erstreckende Punktionsnadel führt, als auch gleichzeitig den Führungskanal für den Katheter bildet. Nach erfolgter Punktion des Gefäßes durch die Punktionsnadel und Rückführung der Punktionsnadel bis hinter die Abzweigstelle des Abzweigabschnitts kann so der Katheter über den Abzweigabschnitt in den länglichen Gehäuseabschnitt eingeführt werden, wobei entlang des Abzweigabschnitts bis zur Spitze des einteiligen länglichen Gehäuseabschnitts zumindest eine Sollbruchstelle angeordnet ist, die eine Öffnung des Gehäuses ermöglicht, so dass der Katheter nach erfolgter Öffnung freigelegt ist und das Katheterpunktionsbesteck entfernt werden kann.

So umfasst das erfindungsgemäße Katheterpunktionsbesteck in einem einzigen Gehäuse zunächst die Punktionsnadel, die an einem Ende aus dem länglichen Gehäuseabschnitt herausgeschoben wird und das Gefäß punktiert. Dabei wird auch das Ende des Gehäuses selbst ein wenig in das punktierte Gefäß mit eingeschoben. Wurde das Gefäß punktiert, tritt Blut in die Punktionsnadel ein, welches in einem am anderen Ende der Punktionsnadel angeordneten, vorzugsweise durchsichtigen Blutauffangbehälter aufgenommen wird.

Nach erfolgter Punktion wird die Punktionsnadel mit dem daran befindlichen Blutauffangbehälter aus dem Gefäß zurückgezogen, wobei das Gehäuse selbst im Gefäß verbleibt.

Die Punktionsnadel wird so weit zurückgezogen, bis die Spitze hinter der Abzweigstelle des Abzweigabschnitts des Gehäuses zu liegen kommt und ein an der Punktionsnadel angeordneter Anschlag an einem entsprechenden Gegenlager im Gehäuse anschlägt, damit die Punktionsnadel nicht vollständig aus dem Katheterpunktionsbesteck herausgezogen werden kann. Um ein erneutes Einschieben der Punktionsnadel ebenfalls sicher zu vermeiden, sichert ein Sperrelement die Punktionsnadel, so dass deren mit Blut verunreinigte Spitze sicher im Gehäuse zu liegen kommt und keine Verletzungsgefahr für das medizinische Personal oder anderen Personen darstellt.

In der erfinderischen Bauform, wird dies durch einen Spreizkörper erreicht, dessen Fangarme sich nach überschreiten einer Absatzstufe innenseitig im rohrartigen Gehäuse einspreizen und so ein nach vorne Schieben der Punktionsnadel abschließend verhindern. Diese Arretierung ist nicht aufhebbar.

Dieser erfindungsgemäße Spreizkörper mit Fangarmen ist vorderseitig an der Punktionsnadel befestigt, wobei die Ausrichtung der Fangarme bewirkt, dass ein Rückführen der Punktionsnadel ohne weiteres möglich ist, da die Fangarme sich zwar an den inneren Mantel des rohrartigen Gehäuses anlegen, durch die Zugbewegungsrichtung allerdings keine Blockierung der Bewegung erfolgen kann. Erst nach Einrasten in der entsprechenden Gehäusestufe können sich die Fangarme weiter aufspreizen und so die Arretierung bewirken.

Anschließend wird der Katheter über den Abzweigabschnitt durch das Gehäuse in das punktierte Gefäß eingeführt und soweit in das Gefäß hineingeschoben wie gewünscht.

Als letzter Arbeitsschritt werden nun die beiden am Abzweigabschnitt sowie am Gehäuse jeweils seitlich verlaufenden Sollbruchstellen mittels einer Aufreißlasche am Abzweigabschnitt geöffnet und beide Gehäuseabschnitthälften vollständig voneinander getrennt.

Der eingeführte Katheter liegt danach frei und das erfindungsgemäße Katheterpunktionsbesteck kann mitsamt Punktionsnadel und Blutauffangbehälter einfach und ohne ein Verletzungsrisiko entsorgt werden.

In einer vorteilhaften Ausführungsform der Erfindung wir der Katheter bis zu seinem Einschieben in das punktierte Gefäß an einer zusätzlich am Gehäuse angeordneten Halterung lösbar fixiert. Es ist hierbei zweckmäßigerweise eine Klemmhalterung vorgesehen, die einhändig zu lösen ist. So ist zum einen sichergestellt, dass bei der eigentlichen Punktierung der flexible Katheter nicht hinderlich ist oder aus dem Punktionsbesteck herausgezogen wird. Zum anderen kann dieser aber nach erfolgter Punktierung leicht aus der Fixierung gelöst und so in das punktierte Gefäß eingeschoben werden.

Eine weitere vorteilhafte Ausgestaltung der Erfindung sieht zwei in etwa parallel verlaufende Sollbruchstellen vor, die sich entlang des Abzweigabschnitts bis zur Spitze des Gehäuses erstrecken. Es ist hierbei vorteilhaft, dass deren Abstand zueinander die Breite eines vom Gehäuse entfernbaren Öffnungsstreifens definiert, wobei diese Breite in etwa der des freizulegenden Katheters entspricht.

So ist sichergestellt, dass das Abziehen des Öffnungsstreifens eine schlitzförmige Öffnung über die gesamte Führungslänge des Katheters im Gehäuse freilegt, so dass der Katheter ohne Kraftaufwand aus dem Gehäuse gelöst werden kann. Dies ist eine wesentliche Verbesserung insofern, dass auf diese Weise sicher verhindert werden kann, dass beim Ablösen des Gehäuses vom in das Gefäß des Patienten eingeschobenen Katheter dieser unbeabsichtigt wieder aus dem Gefäß herausgezogen wird.

Für die Entfernung dieses Öffnungsstreifens ist weiterhin in einer zweckmäßigen Bauform eine daran im Bereich des Abzweigabschnitts angeordnete Aufreißvorrichtung vorgesehen. Dies ist insofern vorteilhaft, da so die Öffnung des Gehäuses entlang der Sollbruchstellen hin zum punktierten Patienten erfolgt und nicht von diesem weg. So wird wiederum sichergestellt, dass der eingeführte Katheter nicht unbeabsichtigt aus dem Gefäß gezogen wird, da die Zugbewegung am Gehäuse zur Entfernung des Öffnungsstreifens hin zum Patienten erfolgt.

Es ist hierbei bei einer vorteilhaften Ausgestaltung des Katheterpunktionsbestecks eine Aufreißvorrichtung an der Sollbruchstelle vorgesehen, die als zumindest einseitige Greiffläche zur besseren Zugkrafteinleitung in die Sollbruchstelle ausgebildet ist. Durch Ergreifen dieser vorsprungartigen Greiffläche, die in ihrer Form und Größe auf ein sicheres Halten beispielsweise zwischen Zeigefinger und Daumen hin gestaltet ist, kann die benötigte Kraft zur Überwindung des Bruchwiderstandes der beispielsweise gekerbt ausgebildeten Sollbruchstelle sicher eingeleitet werden.

In den Figuren 1 bis 4 wird zur näheren Beschreibung eine erfindungsgemäße Ausführungsform des einteiligen Katheterpunktionsbestecks ausführlich dargestellt.

Es zeigen
- Figur 1: eine perspektivische Darstellung eines erfindungsgemäßen Katheterpunktionsbestecks etwa in Originalgröße,
- Figur 2: eine Ausschnittsvergrößerung einer Schnittansicht durch das Gehäuse nach erfolgter Punktion mit zurückgezogener Punktionsnadel,
- Figur 3: eine perspektivische Darstellung des Katheterpunktionsbestecks beim Öffnen der Sollbruchstellen und
- Figur 4: das Besteck nach erfolgter vollständiger Öffnung der Sollbruchstellen.

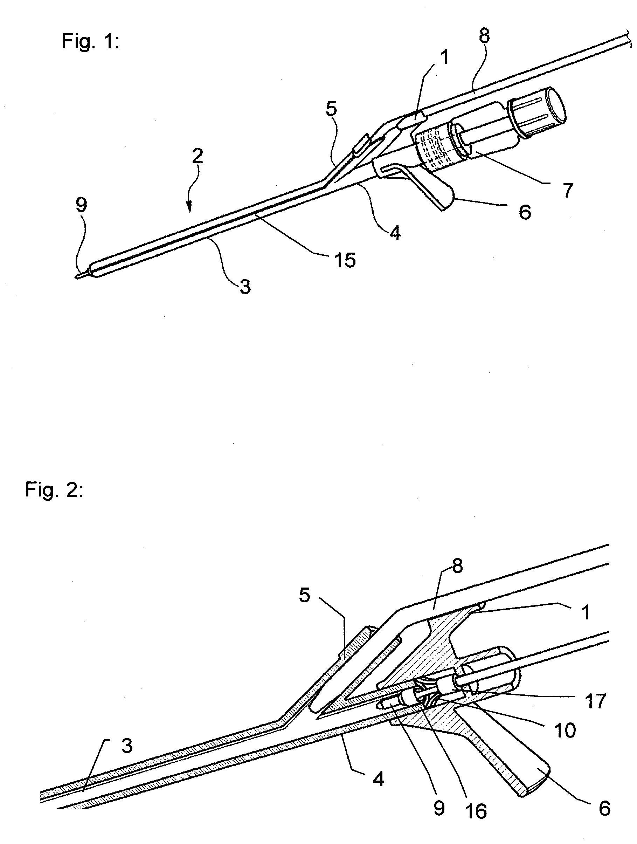

Figur 1 zeigt das erfindungsgemäße Katheterpunktionsbesteck 2 bestehend aus einem rohrartigen Gehäuse 3 mit einem länglichen Gehäuseabschnitt, der in einen Verlängerungsabschnitt zur Führung der Punktionsnadel 9 mit Blutauffangbehälter 7, Anschlag und Sperrelement 17 übergeht, und von dem unter einem Winkel ein Abzweigabschnitt 5 abzweigt, über welchen ein Katheter 8 in den länglichen Gehäuseabschnitt einschiebbar ist. In diesen baulichen Eigenschaften folgt das neue Katheterpunktionsbesteck dem Stand der Technik.

Das erfindungsgemäße Katheterpunktionsbesteck 2 ist in seiner äußeren Erscheinung bereits deutlich schmaler gehalten, als dies bei den im Stand der Technik zitierten Bauformen der Fall war. Dies wird primär dadurch realisiert, dass die Sollbruchstellen 15, die in der dargestellten Bauform lediglich einseitig erkennbar sind, bei der konkreten baulichen Lösung aber auch auf der nicht erkennbaren Rückseite der Vorrichtung parallel verlaufen, es ermöglichen, dass sowohl der Katheter 8 als auch die Kanüle 9 zur Punktion des Gefäßes in demselben rohrartigen Gehäuse 3 geführt werden können.

Es soll an dieser Stelle erwähnt sein, dass der Blutauffangbehälter 7, der über eine Schraubverbindung am hinteren Gehäuseabschnitt 4 mit Handgriff 6 angeordnet ist, nicht zwingend in dieser dargestellten Bauform vorliegen muss. Alternativ kann der Blutauffangbehälter 7 auch durch eine aufgesteckte Einwegspritze, beispielsweise eine 10 ml Spritze, ersetzt werden, ohne dass die bauliche Funktionalität hierdurch beeinträchtigt ist. Insofern stellt der Blutauffangbehälter 7 in der hier dargestellten baulichen Lösung lediglich eine mögliche Bauform vor. Als Blutauffangbehälter 7 ist grundsätzlich jeder Körper zu verstehen, der auf das Katheterpunktionsbesteck 2 aufgesetzt sein kann und hier ein Austreten des Blutes bei der Punktion verhindert.

Betrachtet man nun Figur 2, die eine Schnittdarstellung des Katherterpunktionsbestecks 2 im Bereich der Zufuhr des Katheters 8 über den Abzweigabschnitt 5 darstellt, so wird diese Doppelfunktion des rohrartigen Gehäuses 3 des Katheterpunktionsbestecks 2 sehr deutlich. Es ist hier der Verfahrensabschnitt in Figur 2 dargestellt in dem das Katheterpunktionsbesteck 2 bereits erfolgreich in ein Gefäß des Patienten eingeführt worden ist, was hier bildlich nicht dargestellt ist. Nach dieser Punktion kann die Kanüle 8 bis hinter den Abzweigabschnitt 5 in einen hinteren Bereich 4 des Katheterpunktionsbestecks 2 zurückgezogen werden, wo eine Arretierung erfolgt. Diese Arretierung bewirkt, dass diese Punktionsnadel 9 weder nach vorne noch weiter aus dem Besteck heraus bewegt werden kann.

In der erfinderischen Bauform, die in Figur 2 dargestellt ist, wird dies durch einen Spreizkörper 17 erreicht, dessen Fangarme 10 sich nach überschreiten einer Absatzstufe 16 innenseitig im rohrartigen Gehäuse 3 einspreizen und so ein nach vorne Schieben der Punktionsnadel 9 abschließend verhindern. Diese Arretierung ist nicht aufhebbar.

Dieser erfindungsgemäße Spreizkörper 17 mit Fangarmen 10 ist vorderseitig an der Punktionsnadel 9 befestigt, wobei die Ausrichtung der Fangarme 10 bewirkt, dass ein Rückführen der Punktionsnadel 9 ohne weiteres möglich ist, da die Fangarme 10 sich zwar an den inneren Mantel des rohrartigen Gehäuses 3 anlegen, durch die Zugbewegungsrichtung allerdings keine Blockierung der Bewegung erfolgen kann. Erst nach Einrasten in der entsprechenden Gehäusestufe 16 können sich die Fangarme 10 weiter aufspreizen und so die Arretierung bewirken.

Figur 2 zeigt zudem das vordere Ende der einzuführenden Kanüle 8, welches im Abzweigabschnitt 5 des Katheterpunktionsbestecks 2 eingeschoben ist. In der Zusammenschau mit Figur 1 ist erkennbar, dass die Fixierung des Katheters 8 in dieser Ausgangsposition erreicht wird durch eine Halterung 1, die den Katheter 8 so lange in dieser Position fixiert, bis dieser in das punktierte Gefäß eingeschoben werden soll. Es ist hierfür vorgesehen, den Katheter 8 zum weiteren Einschieben aus der Klemmhalterung 1 nach oben heraus zu ziehen und somit in eine bewegliche Position zu versetzen. Da nach erfolgter Punktion die Punktionsnadel 9 nicht mehr das rohrartige Gehäuse 3 als Führung für den Katheter 8 versperrt, findet sich ein offener Führungskanal, durch den der Katheter 8 nun in das Gefäß des Patienten eingeführt werden kann.

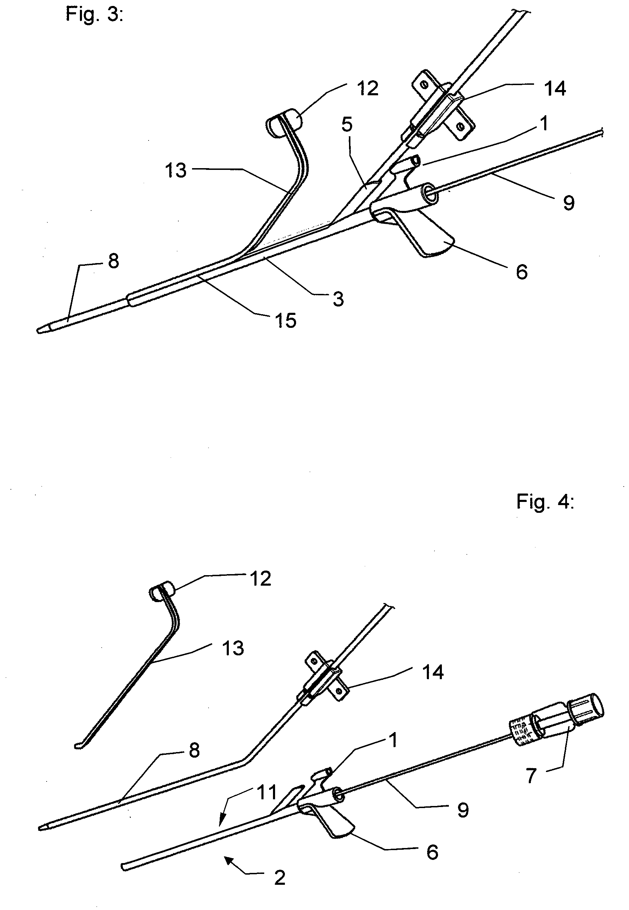

Figur 3 zeigt, wie nach dem Einschieben des Katheters 8 in das punktierte Patientengefäß weiter verfahren wird. Da der Katheter 8 nun gelegt ist, kann das rohrartige Gefäß 3 aus dem punktierten Gefäß des Patienten herausgezogen werden. Allerdings ist der Katheter 8 noch in dem rohrartigen Gehäuse 3 des Katheterpunktionsbestecks 2 eingeschlossen und muss erst aus diesem herausgelöst werden. Hierfür ist ein Ablösen eines Öffnungsstreifens 13 vorgesehen, der durch die zwei in Längsrichtung von der Abzweigstelle 5 zur Spitze des rohrartigen Gehäuses 3 verlaufenden Sollbruchstellen 15 definiert wird.

Figur 3 zeigt hierbei den Vorgang des Ablösens dieses Öffnungsstreifens 13, wodurch wie in Figur 4 dargestellt die einzelnen Bestandteile des Katheterpunktionsbestecks 2 aufgetrennt werden. Das vormals einteilig ausgebildete Katheterpunktionsbesteck 2 zerfällt nach dem Abziehen des Öffnungsstreifens 13 in den eigentlichen gelegter Katheter 8, das Katheterpunktionsbesteck 2 mit vollständig bis zum Anschlag herausgezogener Punktionsnadel 9 und länglicher Austrittsöffnung 11 für den Katheter sowie den Öffnungsstreifen 13, der die entsprechende Öffnung 11 freigelegt hat.

Es wird hierbei deutlich, dass die Punktionsnadel 9 sicher im Katheterpunktionsbesteck 2 verbleibt und auch das bei der Punktion ausgetretene Blut sicher im Blutauffangbehälter 7 verbleibt, ohne dass eine Kontamination des mit dem Katherpunktionsbesteck 2 arbeitenden Personal zu befürchten ist. Insofern erfüllt die vorliegende Sicherheitskanüle mit Arretierung die entsprechenden Anforderungen des Medizinproduktegesetzes.

Die Ablösung des Öffnungsstreifens 13 an den parallel verlaufenden Sollbruchstellen 15 entlang des rohrartigen Gehäuses 3 wird erleichtert durch Greifflächen 12 im Bereich des Abzweigabschnitts 5. Diese zu beiden Seiten des Katheterpunktionsbestecks 2 abzweigenden flügelartigen Greifflächen 12 ermöglichen ein leichtes Ergreifen dieses Bereichs beispielsweise zwischen Daumen und Zeigefinger, wodurch die Gefahr eines unbeabsichtigten Herausziehen des bereits im Gefäß befindlichen Katheters 8 weiter reduziert werden kann.

## Patentansprüche

1. Katheterpunktionsbesteck (2) umfassend ein einteilig und geschlossen ausgebildetes rohrartiges Gehäuse (3) mit einem länglichen Gehäuseabschnitt, der in einen Verlängerungsabschnitt zur Führung der mit ihrer Spitze aus dem länglichen Gehäuseabschnitt vorderseitig austretende Punktionsnadel (9) mit Blutauffangbehälter (7), Anschlag und Sperrelement übergeht, und von dem unter einem Winkel ein Abzweigabschnitt (5) abzweigt, über welchen ein Katheter (8) nach erfolgter Punktion und Rückführung der Punktionsnadel (9) bis hinter die Abzweigstelle des Abzweigabschnitts (5) in den länglichen Gehäuseabschnitt einschiebbar ist, wobei entlang des Abzweigabschnitts (5) bis zur Spitze des länglichen Gehäuseabschnitts zumindest eine Sollbruchstelle (15) verläuft, mithilfe derer das rohrartige Gehäuse (3) des Katheterpunktionsbestecks (2), nachdem der Katheter (8) hindurch geschoben wurde, aufgebrochen werden kann, um das Gehäuse (3) vom Katheter (8) zu entfernen, wobei das Sperrelement die Punktionsnadel (9) nach dessen Rückführung über der Abzweigstelle des Abzweigabschnitts (5) fixiert,
**dadurch gekennzeichnet, dass**
das Sperrelement als Spreizkörper (17) im vorderen Abschnitt der Punktionsnadel (9) ausgebildet ist, dessen Fangarme (10) beim Zurückziehen der Punktionsnadel (9) über den Abzweigabschnitt (5) hinaus in eine Absatzstufe (16) im hinteren Bereich (4) des rohrartigen Gehäuses (3) einspreizen und die Punktionsnadel so arretieren.

2. Katheterpunktionsbesteck nach Anspruch 1,
**dadurch gekennzeichnet, dass**
am Gehäuse (3) mindestens eine Halterung (1) angeordnet ist, die den Katheter (8) bis zur Einschiebung desselben in das Gehäuse (3) im Abzweigabschnitt (5) lösbar fixiert.

3. Katheterpunktionsbesteck nach Anspruch 1 oder 2,
**dadurch gekennzeichnet, dass**
die Spitze des länglichen Gehäuseabschnitts, aus welchem die Punktionsnadel (9) zur Punktion austritt, spitz gerundet ausgebildet ist.

4. Katheterpunktionsbesteck nach einem der vorangegangenen Ansprüche,
**dadurch gekennzeichnet, dass**
die mindestens eine Sollbruchstelle (15) mittels einer daran angeordneten Aufreißvorrichtung (12) versehen ist.

5. Katheterpunktionsbesteck nach Anspruch 4, **dadurch gekennzeichnet, dass**
die Aufreißvorrichtung (12) an der Sollbruchstelle (15) als zumindest eine Greiffläche zur besseren Zugkrafteinleitung in die zumindest eine Sollbruchstelle (15) ausgebildet ist.

6. Katheterpunktionsbesteck nach einem der vorangegangenen Ansprüche,
**dadurch gekennzeichnet, dass**
zwei in etwa parallel verlaufende Sollbruchstellen (15) sich entlang des Abzweigabschnitts (5) bis zur Spitze des Gehäuses (3) erstrecken, deren Abstand zueinander die Breite eines vom Gehäuse (3) entfernbaren Öffnungsstreifens (13) definiert, wobei diese Breite in etwa der des freizulegenden Katheters (8) entspricht.

## Claims

1. Catheter puncture device (2) comprising a tubular housing (3) which is in one piece and of a closed design with an elongate housing portion, which merges into an extension portion for guiding the puncture needle (9), protruding with its tip from the front of the elongate housing portion, with blood collection container (7), abutment and blocking element, and from which a branch portion (5) branches off at an angle, through which branch portion (5) a catheter (8) can be pushed into the elongate housing portion after the puncture has been made and the puncture needle (9) has been returned to a point behind the branching site of the branch portion (5),
wherein at least one predetermined breaking point (15) extends along the branch portion (5) as far as the tip of the elongate housing portion, which predetermined breaking point (15) allows the tubular housing (3) of the catheter puncture device (2) to be broken open in order to remove the housing (3) from the catheter (8) after the catheter (8) has been pushed through, wherein the blocking element fixes the puncture needle (9) after said blocking element has been returned through the branching site of the branch portion (5),
**characterized in that**
the blocking element is designed as an expansion body (17) in the front portion of the puncture needle (9) and, when the puncture needle (9) is pulled back past the branch portion (5), the catching arms (10) of the expansion body (17) spread open into a step (16) in the rear area (4) of the tubular housing (3) and thus lock the puncture needle.

2. Catheter puncture device according to Claim 1,
**characterized in that** at least one holder (1) is arranged on the housing (3) and releasably fixes the catheter (8) in the branch portion (5) until said catheter (8) is pushed into the housing (3).

3. Catheter puncture device according to Claim 1 or 2, **characterized in that** the tip of the elongate housing portion, from which the puncture needle (9) emerges to perform the puncture, is rounded to a point.

4. Catheter puncture device according to one of the preceding claims, **characterized in that** the at least one predetermined breaking point (15) is provided by means of a tearing-open device (12) arranged thereon.

5. Catheter puncture device according to Claim 4,
**characterized in that** the tearing-open device (12) on the predetermined breaking point (15) is designed as at least one grip surface for better introduction of tensile force into the at least one predetermined breaking point (15).

6. The catheter puncture device according to one of the preceding claims, **characterized in that** two approximately parallel predetermined breaking points (15) extend along the branch portion (5) as far as the tip of the housing (3), the distance between them defining the width of an opening strip (13) removable from the housing (3), wherein this width corresponds approximately to that of the catheter (8) that is to be exposed.

## Revendications

1. Ensemble de ponction pour cathéter (2) comprenant un boîtier tubulaire formé en une seule pièce et fermé (3) avec une partie de boîtier allongée, qui se prolonge en une partie de prolongement pour le guidage de l'aiguille de ponction (9) sortant avec sa pointe à l'avant hors de la partie de boîtier allongée avec un récipient de collecte de sang (7), une butée et un élément d'arrêt, et de laquelle part sous un certain angle une partie de dérivation (5) par laquelle un cathéter (8) peut être glissé dans la partie de boîtier allongée après l'exécution de la ponction et le retrait de l'aiguille de ponction (9) jusque derrière le point de branchement de la partie de dérivation (5), dans lequel une zone de rupture théorique (15) s'étend le long de la partie de dérivation (5) jusqu'à la pointe de la partie de boîtier allongée, à l'aide de laquelle le boîtier tubulaire (3) de l'ensemble de ponction pour cathéter (2) peut être rompu, après que le cathéter (8) ait été inséré, afin d'enlever le boîtier (3) du cathéter (8), dans lequel l'élément d'arrêt fixe l'aiguille de ponction (9) après son retrait au-delà du point de branchement de la partie de dérivation (5), **caractérisé en ce que** l'élément d'arrêt est réalisé sous la forme d'un corps expansible (17) dans la partie avant de l'aiguille de ponction (9), dont les bras de saisie (10) s'écartent lors du retrait de l'aiguille de ponction (9) au-delà de la partie de dérivation (5) dans un épaulement (16) dans la région arrière (4) du boîtier tubulaire (3) et bloquent de ce fait l'aiguille de ponction.

2. Ensemble de ponction pour cathéter selon la revendication 1, **caractérisé en ce qu'**au moins un support (1) est disposé sur le boîtier (3), et fixe de façon séparable le cathéter (8) dans la partie de dérivation (5) jusqu'à l'insertion de celui-ci dans le boîtier (3).

3. Ensemble de ponction pour cathéter selon la revendication 1 ou 2, **caractérisé en ce que** la pointe de la partie de boîtier allongée, de laquelle l'aiguille de ponction (9) sort pour une ponction, est de forme arrondie tranchante.

4. Ensemble de ponction pour cathéter selon l'une quelconque des revendications précédentes, **caractérisé en ce que** ladite au moins une zone de rupture théorique (15) est munie d'un dispositif de déchirure (12) disposé sur celle-ci.

5. Ensemble de ponction pour cathéter selon la revendication 4, **caractérisé en ce que** le dispositif de déchirure (12) est formé sur la zone de rupture théorique (15) sous la forme d'au moins une face de prise pour une meilleure application d'une force de traction à la zone de rupture théorique (15).

6. Ensemble de ponction pour cathéter selon l'une quelconque des revendications précédentes, **caractérisé en ce que** deux zones de rupture théorique sensiblement parallèles (15) s'étendent le long de la partie de dérivation (5) jusqu'à la pointe du boîtier (3), dont la distance l'une de l'autre définit la largeur d'une bande d'ouverture (13) pouvant être enlevée du boîtier (3), dans lequel cette largeur correspond environ à celle du cathéter (8) à libérer.
